(19) Europäisches Patentamt
European Patent Office
Office européen des brevets

(11) EP 1 138 758 B1

(12) EUROPEAN PATENT SPECIFICATION

(45) Date of publication and mention
of the grant of the patent:
17.01.2007 Bulletin 2007/03

(51) Int Cl.:
C12M 1/34 (2006.01)          G01N 33/487 (2006.01)
G01N 27/22 (2006.01)

(21) Application number: 01500079.7

(22) Date of filing: 26.03.2001

(54) **Method for measuring the concentration and composition of biomass, probe and cell**

Sonde, Zelle und Verfahren zur Messung der Konzentration und der Zusammensetzung von Biomasse

Sonde, cellule et procédé de mesure de la concentration et de la composition d'une biomasse

(84) Designated Contracting States:
AT BE CH CY DE DK ES FI FR GB GR IE IT LI LU
MC NL PT SE TR

(30) Priority: 27.03.2000 ES 200000745

(43) Date of publication of application:
04.10.2001 Bulletin 2001/40

(73) Proprietor: NTE, S.A.
08186 Lliça d'Amunt (Barcelona) (ES)

(72) Inventors:
• Elvira Canas, Jordi
  08014 Barcelona (ES)
• Rosell Ferrer, Javier
  08034 Barcelona (ES)
• Riu Costa, Pere
  08034 Barcelona (ES)
• Bragos Bardia, Ramon
  08034 Barcelona (ES)
• Casas Piedrafita, Oscar
  08034 Barcelona (ES)

(74) Representative: Davila Baz, Angel
c/o Clarke, Modet & Co.,
Goya, 11
28001 Madrid (ES)

(56) References cited:
EP-A- 0 028 793          EP-A- 0 277 789
EP-A- 0 319 198          EP-A- 1 046 905
WO-A-94/03583            GB-A- 2 247 530
GB-A- 2 329 711          US-A- 5 981 268

• PATENT ABSTRACTS OF JAPAN vol. 014, no. 183
(P-1035), 12 April 1990 (1990-04-12) & JP 02
031148 A (KOBE STEEL LTD), 1 February 1990
(1990-02-01)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

**Description**

The object of the present invention is improvements made to Spanish Patent 2143911, which relates to a method and device for measuring the concentration and composition of biomass, which allows to measure low concentrations of said biomass and the concentration and composition of biomass in active sludge.

Biomass measurement systems are previously known, but which have problems when measuring at low concentrations. This is because the measurements they perform are very sensitive to changes in the temperature and conductivity of the medium in which the biomass is cultivated. At low concentrations the signal is very low and its sensibility to electronic noise implies that measurements cannot be correctly performed.

This problem is solved by the method described in Patent ES 2143911, by the same applicants, according to which the measurement procedure begins with the digital generation of the electric current, although this can also be performed analogically, and its injection into the culture medium with two electrodes. By means of another two electrodes the voltage drop across the medium is measured. This provides a four-wire measurement which to a great extent makes the result independent from measurements of the contact impedance of the electrodes with the medium. The signal amplifiers are placed very near the probe so that parasitic capacitances do not affect the signal, as in certain moments high frequencies are used. The relationship between the voltage and current signals is the electrical impedance. This process is repeated for several frequencies between 1 kHz and 20 MHz. The frequencies can be selected depending on the type of biological culture involved. The electrical impedance is constantly corrected for the aforementioned measurements of temperature and conductivity using known linear coefficients.

From the results obtained two data processes may be carried out which lead to estimation parameters for biomass or cell concentration:

1) The relationship between low and high frequency impedances is proportional to the cell concentration in the medium. Previously, the measurement device is calibrated for the type of cells indicated. The relationship between the high and low values provides a good estimate:

[1]

$$E(\%) = \frac{|Z(BF)| - |Z(AF)|}{|Z(BF)|} \cdot 100$$

where $|Z(AF)|$ and $|Z(BF)|$ are the modulus of the impedances measured at high and low frequencies respectively.

2) Measurements of electrical impedance resulting from frequency scanning ( at least four measurements between 1 kHz and 20 MHz) are fitted to a curve:

[2]

$$Z(f) = R_\infty + \frac{R_0 - R_\infty}{1 + (j\frac{f}{f_c})^{1-\alpha}}$$

using a least squares method. Parameters $R_0$ and $R\infty$ are the resistances shown by the medium in direct current and at very high frequency respectively; $f_c$ is the frequency at which the imaginary part maximum occurs, which depends on the type of microorganism; and $\alpha$ is a parameter related to the dispersion of cell sizes and shapes of the microorganisms.

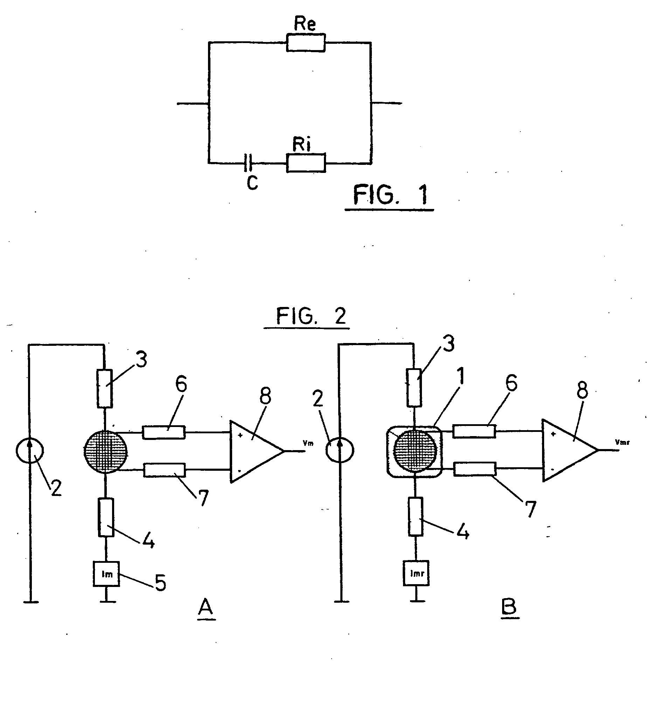

The impedance measured may be modeled by the circuit shown in figure 1. The relationship between the circuit parameters (Re, resistance of the extra-cellular medium and Ri, intracellular resistance) and those of the mathematical model ($R_0$ and $R\infty$) is as follows:

$$R_e = R_0$$

[3]

$$R_i = \frac{R_0 \cdot R_\infty}{R_0 - R_\infty}$$

$$[4] \qquad E(\%) = \frac{R_0 - R_\infty}{R_0} \cdot 100$$

where $R_0$ and $R_\infty$ are obtained from the aforementioned fit of the equation modeling the variation of impedance with frequency. The relationship:

$$[5] \qquad E(\%) = \frac{R_0 - R_\infty}{R_0} \cdot 100$$

gives an estimate of the concentration of biomass. This relationship is the same as in option 1 of the data processing but with values obtained from fitting to the model.

In short, the two options (formulas 1 and 5) allow to obtain a good estimate for the biomass through the relationship between the high and low frequency impedances. One of the two options directly employs the values measured at high and low frequency and the other uses values obtained from fitting impedance data from a frequency scan to a model.

The second option (formula 5) also provides two additional parameters ($f_c$ and $\alpha$) which provide information on the cells of the system, allowing to determine the type of cell involved as well as the dispersion of sizes. The system could also detect whether the culture is contaminated with cells of a different type.

Final data obtained by the measurement device are processed by a computer and can present the user with constant information of the cell concentration in the medium.

However, this procedure has its limitations when operating at low concentrations. The main limitation of the above described estimations is that they assume the conductivity of the extra-cellular medium ($\sigma_e$) and that of the intracellular medium ($\sigma_i$) to be similar and constant. The minor variations which $\sigma_e$ undergoes in a culture are not critical for estimating biomass density if the latter is quite high, but they may mask measurements of low concentrations. In this case we must introduce an additional procedure which affects the structure of the measurement.

The present invention proposes to add a further step allowing to reach lower concentrations: this step consists of measuring parameter E (%) using the first or second option (formulae 1 or 5) comparing measurements of a reference medium without cells and of one with cells. The mediums used (with and without cells) are identical except for the presence of cells.

The measurement performed in the cell-free medium provides information on the variations of $\sigma_e$ and allows to correct estimator E(%). This can be performed in two ways:

1) Using an expression obtained from the theoretical model:

$$[6] \qquad E_c(\%) = E(\%) \cdot 3 \frac{k}{2+k} \; ; \; k = \frac{\sigma_i}{\sigma_e}$$

$\sigma_i$ may be considered as constant for a given microorganism.

2) Using a calibration curve obtained from experimental measurements where $\sigma_e$ is varied artificially.

If additionally the reference measurement is taken with a set of electrodes which is identical in materials, size and shape to that used for measuring the biomass density, it is possible to cancel effects related to the non ideality of amplifiers, connection cables and electrodes, as well as to their variation with temperature.

Comparison and/or processing of measurements made with both sets of probes can be performed in two manners:

1) Individual measurements in each probe and comparison using software. Each individual measurement can be performed with a frontal stage for each probe or by multiplexing a single frontal stage between the two probes.
2) Direct differential measurements of the voltages of detection electrodes in both probes by connection in a Wheatstone bridge adapted to 4 wire impedances (Kelvin bridge) or similar structure.

A further object of this invention is providing the same conditions for the cell-fee and the cell-containing mediums. For this purpose a probe is designed having two measurement regions identical in size. The probe is introduced in the culture for online measurement of biomass, i.e. for measuring directly during the process without requiring to extract any culture samples. One of the regions is covered by a mesh which prevents entry of the cell of interest (mesh orifice diameter smaller than smallest radius of the cell measured) while the other is in direct contact with the cell containing culture. The comparison is made between the measurements of these two regions.

For measurements of lower concentrations off-line measurements are required. This is performed as follows: 1) A sample of the culture is taken and divided into two equal samples which are introduced in two identical containers with two sets of 4 identical electrodes. 2) One of the containers is subjected to ultrasound waves which cause the lysis of cells in the culture. As an alternative the lysis may be caused by a chemical, electric or thermal process. 3) With this last container as the reference container, the two containers are set at the same temperature, such as by placing them in an array at constant temperature, and the measurement is performed by comparing the concentration of cells in the culture.

In cultures where cells are grown in layers adhered to a surface an assembly is made of two sets of 4 identical electrodes with each set in 2 identical regions, but with one of the sets mounted at a region which is not biocompatible and does not allow cell growth in the region where the electrodes are measuring.

With the system described, and by the two above procedures, measurement of low concentrations of biomass is made feasible (such as for Saccharomyces cerevisiae in concentrations under $1 \times 10^6$ cells per milliliter).

The improvements object of the present invention also allow to measure the biomass present in active sludge. Active sludge is grown in biological reactors in urban depurators and in waste treatment plants. In these biological reactors active sludge act by purifying the water contained in the reactor.

Online measurement of biomass contained in active sludge would entail measuring parameter E(%) with respect to its value in the biomass-free liquid of the reactor. Using a suitable probe the required comparison measurement can be performed.

An extension of this procedure would be employed for off-line measurement. Typically these measurements would employ the V-30 measurement standard, which consists of measuring the height of active sludge after filling a vessel to 1000 cc and waiting 30 minutes for sedimentation of the active sludge to take place. A vessel is proposed provided with 4 electrodes in the supernatant part and 4 electrodes in the lower part where the active sludge sediments.

The measurement procedure using said probe is as follows: 1) the liquid which will be a sample of the reactor with the active sludge to be measured is introduced; 2) it is filed to 1000 cc; 3) the rate of sedimentation is measured as follows: Z(f) is measured repeatedly at the lower electrode and the time evolution is analysed of the modulus of impedance at a single frequency, or of estimator E(%). Because of the sedimentation said time evolution will follow an exponential pattern with saturation:

$$[7] \qquad y(t) = y_0 + A \cdot \left( 1 - e^{-\frac{t}{\tau}} \right)$$

Time constant $\tau$ provides information on the rate of sedimentation of the sludge and the waiting time required to obtain a stable measurement of the biomass density estimator. Parameters $y_0$ A, provide information on the properties of the stirred medium and on the sedimented medium respectively; 4) After ensuring that sedimentation has ended, or once $\tau$ can be calculated the electrical impedance is measured by comparing the supernatant and lower part of the sludge as described above, as well as measuring the height of the sedimented medium (h), such as by optical means; 5) A final estimator E(%) is obtained together with characteristics of the biomass being measured ($f_c$ and $\alpha$); 6) These measurements of $f_c$ and $\alpha$ give an idea of the type of cells under study. The system may compare these values to those for other cultures to estimate the generic population distribution.

This method can be considered a variation of the previously described method, the present one characterised in that the microorganism-free area is at the top of the vessel, using the sedimentation property of solids in suspension and biomass present in active sludge. Accumulation of biomass near the lower electrode allows obtaining a measurement farther from the device's noise level than would be possible with a stirred suspension. The relationship between the measurement on the sediment and average biomass density in said stirred suspension can be obtained from the sedimentation level and a calibration curve found experimentally.

The characteristics of the invention will be made clearer by the following description made with reference to the accompanying drawings of a non-limiting example.

In the drawings:

Figure 1 is a schematic representation of a circuit equivalent to the electric model of a biological material measured in a culture medium with the procedure object of principal patent n° 2143911.

Figure 2 is an individual measurement scheme for each probe.

Figure 3 is a schematic representation of the direct differential measurement of the voltages in detector electrodes of both probes.

Figure 4 shows a partially sectioned view of a probe structured to carry out the object of the invention.

Figure 5 is a schematic representation of the concentrations measurement using independent containers. Figures 6 and 7 are a lateral and plan view of a vessel used to carry out the procedure of the invention.

Figure 8 is a schematic representation of an alternative concentration measurement system.

Figure 9 is a comparison diagram for the characteristic values of the biomass of different cultures.

Figure 1 shows an equivalent circuit for the measurements obtained in a culture medium with the procedure object of the principal patent 2143911. When the frequency of the injected current is very high capacitor C behaves as if it were shunted and the entire current flows through the intracellular medium with an electrical resistance $R_i$ and through the extra-cellular medium with a resistance $R_e$. When the frequency is very low the capacitor C behaves as if it were in open circuit and forces all the current to pass only through the extra-cellular medium with a resistance $R_e$.

As mentioned above the object of the invention is to obtain measurements of biomass with lower concentrations than those allowed by principal patent 2143911 for which the electrical impedance in the medium analysed is measured in two different regions, one free of cells and the other containing cells. These measurements are performed using probes with sets of identical electrodes.

The comparison and/or processing of measurements performed with the two probes can take place as shown in figure 2, where two regions are shown: region A contains biomass and region B shares the same culture medium with region A but is free of biomass by using any of the procedures described above, such as a filter (1).

In both cases a current is injected in the samples using a generator (2) through electrodes (3) and (4). The current which flows through the samples is measured by electrode (4) using a current measuring circuit (5) which provides measurements Im for the region A and Imr for reference region B.

The voltage drop across both regions is detected using another pair of electrodes (6) and (7), in a differential amplifier (8) which provides measurements Vm for region A and Vmr for reference region B.

The impedance of region A is determined using measurements Vm and Im at different frequencies and the impedance of region B is determined using Vmr and Imr, at these same frequencies.

Differential amplifier (8) and current measurement circuit (5) may be double, one for each region, or single with their connection switchable to the corresponding electrodes when measuring each region.

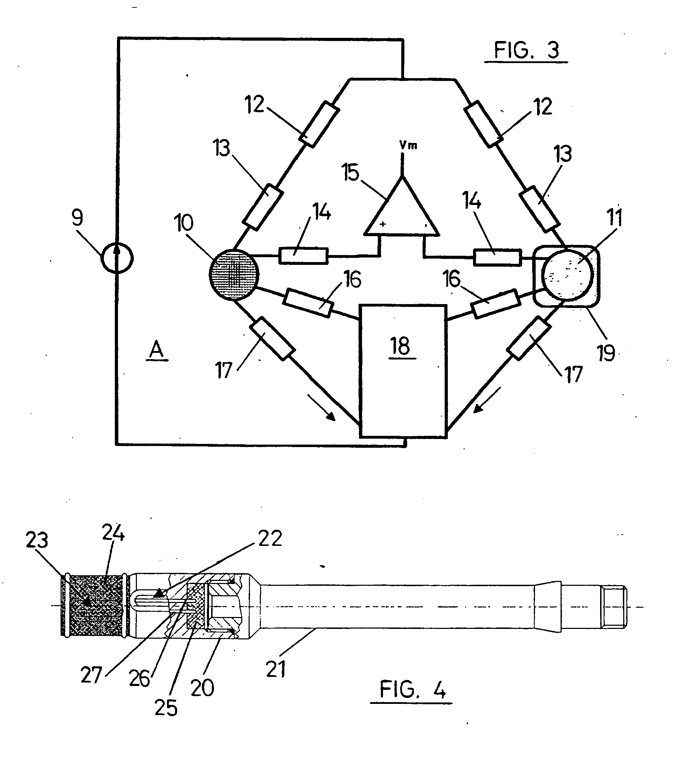

Comparison and processing of the measurements performed with the two probes can also be performed using the direct differential measurement of the voltages at the detector electrodes of each probe as shown in Figure 3.

In this case the current of generator (9) is applied simultaneously to regions (10) and (11) by connection in bridges as shown in the figure. The current divides into two branches through resistors (12) which are connected to an electrode (13) of each region. The voltage drop is measured by another pair of electrodes (14), one connected to each region, by differential amplifier (15). For this difference to be characteristic of the differential behaviour of the two regions, the remaining electrodes (16 and 17) are connected to a current control and measurement circuit (18) which can operate in two modes: a) adjusting and measuring current drained by electrodes (17), so that the voltages in electrodes (16) remain at 0 Volts in both regions; or b) making the two currents of electrodes (17) equal and then measuring the voltages which appear at electrodes (16) of both regions.

In this manner the sensitivity of the system can be increased by directly measuring the differences between regions A and B instead of measuring them separately and then performing a software comparison. Region A is a cell-containing region and region B is an identical but cell-free region, which can be obtained, as in the case of Figure 2, using a filter (19).

Figure 4 shows a biomass measurement probe comprising two parts: base (20) and stem (21). At the base are located two measurement areas, regions (22 and 23), equally sized but with one difference: at region (22) is the biomass which is to be measured while in region (23) a mesh (24) prevents entry of biomass. In area (25) is placed part of the electronics and the electrodes which consist of two sets of four electrodes, (26) and (27).

This probe is introduced in the culture for direct measurement during the process without having to extract any culture samples. Region (23) is covered by mesh (24) which does not allow the cells of interest to pass, for which the orifice diameter of mesh (24) must be smaller than the smallest radius of the cell being studied. Region 22 will be in contact with the cell-containing culture. The comparison is performed between measurements of regions (22) and (23).

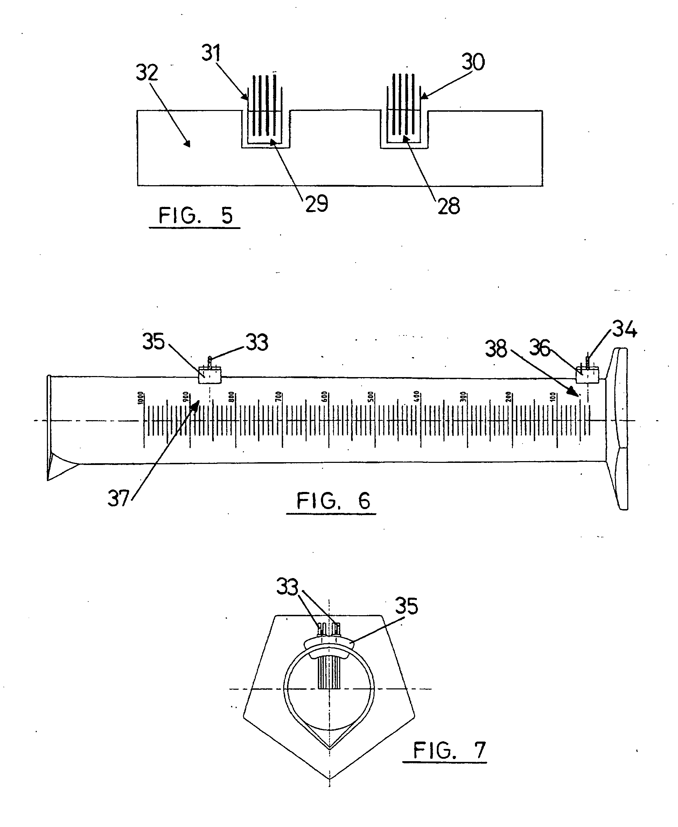

Lower concentrations are measured offline, that is, extracting a culture sample. For this can be used the assembly shown in figure 5, which includes two biomass samples (28) and (29) in two identical vessels (30) and (31). These vessels are placed in a thermostatic block (32). Samples (28) and (29) have one differing property: one is the medium to be measured with the biomass and the other is the medium to be measured with lysed cells, that is with their membranes no longer intact. Vessel (30) may be exposed to ultrasonic waves until the lysis of cells in the culture is achieved. As an alternative, said lysis may be achieved by chemical, electrical or thermal methods.

As indicated above the invention is applicable for measuring biomass present in active sludge. Said measurement may be performed offline, such as by using a measuring tube as shown in figures 6 and 7 with a capacity of 1000 ml in the example of the drawings and which is used to measure sedimentation rate and the biomass present in a 1 liter sample of an active sludge reactor. This measurement tube includes two sets (33) and (34) of four electrodes connected to the tube wall by plastic parts (35) and (36) which are screwed on and hermetised by gaskets. The two sets of electrodes are placed such that one takes readings in region (37), which will include only supernatant when the sludge has sedimented, and one takes readings in region(38) where the active sludge accumulates.

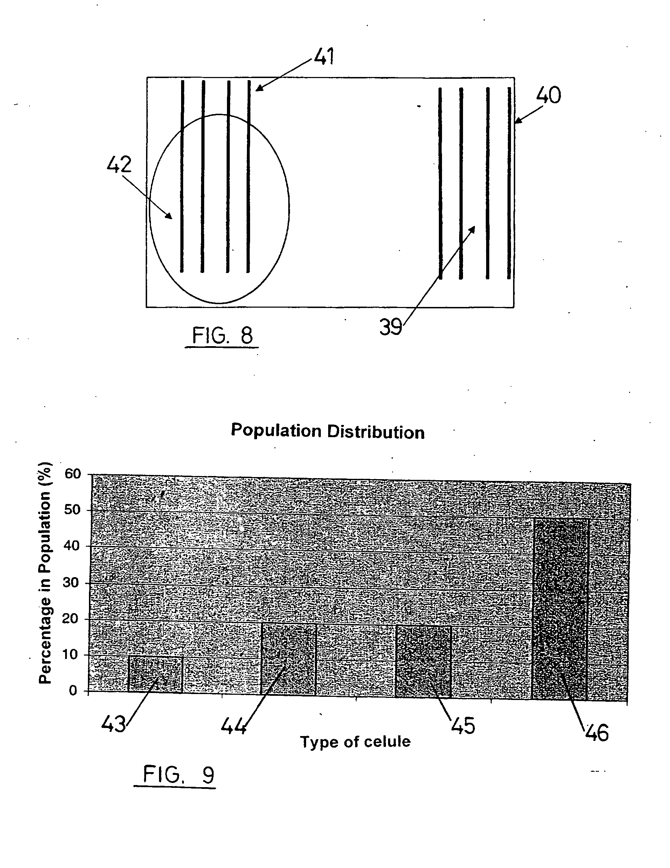

Figure 8 is the view of a surface where measurements are made of a biomass which adheres to said surface and grows as thin layers on top of it (with a thickness on the order of a few cells). On said surface are deposited two sets of a thin layer of a biocompatible metal which may be used as electrodes. These metallic layers must comprise two sets of four electrodes. Region (39) is that formed by the surface of electrodes (40) and their surroundings. In this region (39) cells may grow without any problems. In the surface around the set of electrodes (41) a treatment will be performed so that cells do not grow (such as by placing a non biocompatible material or by treating the surface so that cells cannot adhere to it). The region where biomass cannot grow is region (42). This way there are two regions where the medium may be measured with or without biomass.

In any of the above described embodiments, such as with the measurement tube shown in figures 5 and 7, after the sedimentation has ended the electrical impedance is measured by comparing regions (37) and (38) and a final estimator E(%) is obtained, together with properties of the biomass being measured ($f_c$ and •). These measurements provide information on the type of cell population being studied. This system can compare these values to those of other cultures to provide an estimate of the generic population distribution, as shown in the diagram of figure 9, where the abscissa shows the population distribution and the ordinate shows the population percentage, with this diagram including different cell types such as algae (43), protozoa (44), yeasts (45) and bacteria (46).

## Claims

1. Method for measuring concentration and composition of biomass, which consists of estimating the cellular concentration in the culture by measuring the electrical impedance of the medium in the biomass at several frequencies ranging between 1 kHz and 20 MHz, **characterised in that** the electrical impedance is measured in two regions of the medium, one which is cell-free and the other containing cells, both measurements made with sets of identical electrodes to which are connected two frontal stages of the measurement system; from these measurements of the cell-free area is obtained information on variations in the conductivity of the extra-cellular medium ($\sigma_e$), which information is used to correct the estimate of the cellular concentration of the culture in the cell-containing region.

2. Method as claimed in claim-1, **characterised in that** the correction of the estimate for the cellular concentration based on the variation in conductivity of the extra-cellular medium, $\sigma_e$ , is performed according to the expression:

$$E_c(\%) = E(\%) \cdot 3\frac{k}{2+k} \; ; k = \frac{\sigma_i}{\sigma_e}$$

where the conductivity of the intracellular medium ($\sigma_i$) can be considered constant for a given microorganism.

3. Method as claimed in claim 1, **characterised in that** a correction for the estimate of the cellular concentration is obtained from a calibration curve obtained from experimental data, by artificially varying the conductivity of the extracellular medium ($\sigma_e$).

4. Method as claimed in claim 1, **characterised in that** the cell-free region and the cell-containing region are within the same reactor or culture cell, with the cell-free region separated from the culture by a mesh with a sufficiently narrow opening.

**5.** Method as claimed in claim 1, **characterised in that** the separation between the cell-free region and the cell-containing region is obtained by sedimentation.

**6.** Method as claimed in claim 1, **characterised in that** the cell-free region is obtained by depositing a non-biocompatible substance on the region of reference, or by a treatment which makes this region of reference non-biocompatible.

**7.** Method as claimed in claim 1, **characterised in that** the cell-containing and cell-free regions are obtained from a culture sample which is divided into two equal samples, which are then introduced into identical vessels having identical sets of electrodes, with the cells in one of the samples destroyed by ultra-sound, ultra-violet radiation, chemicals, heat treatment (high temperatures) or by applying high voltage electrical signals.

**8.** Method as claimed in above claims **characterised in that** to compare and/or process the measurements made in the two regions an individual measurement is made in each region using a frontal stage for each set of electrodes, or by multiplexing a single frontal stage between the two sets of electrodes of each region.

**9.** Method as claimed in above claims **characterised in that** to compare and/or process the measurements made with the probes, a direct difference measurement is made of the voltages in the detector electrodes of each probe, by connecting them in a Wheatstone or Kelvin bridge arrangement or the like.

**10.** Method as claimed in claim 5, **characterised in that** sedimentation takes place in a measurement tube in which is introduced a preset volume of sludge, its rate of sedimentation is measured and, during and after the end of sedimentation, its electrical impedance is measured by comparison between the supernatant and active sludge regions, obtaining a final estimator E(%), as well as properties of the biomass being measured according to parameters $f_c$, $y_o$, $\alpha$ and A, which provide information on the type of cells being studied, where: $f_c$ is the frequency at which the maximum imaginary part value is obtained, and which depends on the type of microorganism; $y_o$ gives information on the properties of the stirred medium; $\alpha$ is a parameter related to the dispersion of cell sizes and shapes for the microorganisms; and A gives information about the sedimented medium.

**11.** Method as claimed in previous claims, **characterised in that** from the measurement of the impedance in the two aforementioned medium regions can be obtained, in addition to, estimator E (%), properties of the biomass being measured according to parameters $f_c$, $y_o$, $\alpha$ and A, which provide information on the type of cell population being studied for comparison to values of other cultures, allowing to estimate the generic distribution of the population.

**12.** Probe for measuring biomass in two regions of a bioreactor, **characterised in that** it comprises two measurement regions of equal size, with one region covered by a mesh which does not allow the cells under analysis to pass, while the other region is in direct contact with the cell containing culture.

**13.** Cell for surface cell cultures, **characterised in that** it comprises two sets of measurement electrodes, with the region around one of these sets biocompatible and the region around the other set of electrodes non-biocompatible.

**Patentansprüche**

**1.** Verfahren zur Messung der Konzentration und der Zusammensetzung von Biomasse, welches darin besteht, die Zellkonzentration in der Kultur zu schätzen, indem die elektrische Impedanz des Mediums in der Biomasse bei mehreren Frequenzen im Bereich zwischen 1 kHz und 20 MHz gemessen wird, **dadurch gekennzeichnet, dass** die elektrische Impedanz in zwei Bereichen des Mediums gemessen wird, von denen der eine frei von Zellen ist und der andere Zellen enthält, wobei beide Messungen mit einem Satz gleichartiger Elektroden durchgeführt werden, an welche zwei Frontstufen des Messsystems angeschlossen werden, und wobei aus den Messungen des zellfreien Bereichs Informationen bezüglich der Schwankungen der Leitfähigkeit des extrazellulären Mediums ($\sigma_e$) erhalten werden, welche zur Korrektur der Schätzung über die Zellkonzentration der Kultur im zellhaltigen Bereich verwendet werden.

**2.** Verfahren nach Anspruch 1, **dadurch gekennzeichnet, dass** die Korrektur der Schätzung über die Zellkonzentration, welche auf der Schwankung der Leitfähigkeit des extrazellulären Mediums, $\sigma_e$, basiert, gemäß folgendem Ausdruck durchgeführt wird:

$$E_c(\%) = E(\%) \cdot 3 \frac{k}{2+k} \; ; \; k = \frac{\sigma_i}{\sigma_e}$$

wobei die Leitfähigkeit des intrazellulären Mediums ($\sigma_c$) für einen gegebenen Mikroorganismus als konstant angesehen werden kann.

3. Verfahren nach Anspruch 1, **dadurch gekennzeichnet, dass** die Korrektur der Schätzung über die Zellkonzentration einer Kalibrierkurve entnommen wird, welche in einer Versuchsreihe erstellt wurde, bei der die Leitfähigkeit des extrazellulären Mediums ($\sigma_e$) künstlich Schwankungen unterworfen wurde.

4. Verfahren nach Anspruch 1, **dadurch gekennzeichnet, dass** der zellfreie Bereich und der zellhaltige Bereich innerhalb desselben Reaktors oder desselben Kulturbehälters liegen, wobei der zellfreie Bereich durch ein Netzgewebe mit entsprechend enger Maschenweite von der Kultur getrennt ist.

5. Verfahren nach Anspruch 1, **dadurch gekennzeichnet, dass** die Trennung zwischen dem zellfreien Bereich und dem zellhaltigen Bereich durch Absetzenlassen erzielt wird.

6. Verfahren nach Anspruch 1, **dadurch gekennzeichnet, dass** der zellfreie Bereich **dadurch** geschaffen wird, dass auf den betreffenden Bereich eine biounverträgliche Substanz aufgebracht wird oder dass eine Behandlung durchgeführt wird, welche den betreffenden Bereich biounverträglich macht.

7. Verfahren nach Anspruch 1, **dadurch gekennzeichnet, dass** der zellfreie und der zellhaltige Bereich **dadurch** geschaffen werden, dass eine Probe der Kultur in zwei gleiche Proben geteilt wird, welche anschließend in gleichartige Behälter mit gleichartigen Elektrodensätzen gegeben werden, wobei die Zellen in einer der Proben mittels Ultraschall, ultravioletter Strahlung, Chemikalien, Wärmebehandlung (bei hohen Temperaturen) oder mittels elektrischer Hochspannungssignale zerstört werden.

8. Verfahren nach den vorhergehenden Ansprüchen, **dadurch gekennzeichnet, dass** zum Vergleich und/oder zur Verarbeitung der in den beiden Bereichen vorgenommenen Messungen in jeden Bereich eine Einzelmessung unter Verwendung einer Frontstufe für jeden Elektrodensatz oder unter Verwendung einer Multliplexschaltung zwischen einer einzigen Frontstufe und den beiden Elektrodensätzen der jeweiligen Bereiche durchgeführt wird.

9. Verfahren nach den vorhergehenden Ansprüchen, **dadurch gekennzeichnet, dass** zum Vergleich und/oder zur Verarbeitung der mit Sonden vorgenommenen Messungen eine direkte Differenzmessung der Spannungen in den Detektorelektroden der jeweiligen Sonde durchgeführt wird, indem diese Detektorelektroden miteinander über eine Wheatstonesche Messbrücke oder eine Kelvinbrückenschaltung oder Ähnliches verbunden werden.

10. Verfahren nach Anspruch 5, **dadurch gekennzeichnet, dass** das Absetzenlassen in einer Messröhre erfolgt, in welche ein zuvor festgelegtes Volumen an Schlamm gefüllt wird, woraufhin dessen Absetzrate gemessen und dessen elektrische Impedanz während des Absetzvorgangs und nach dessen Ende durch den Vergleich zwischen dem Überstand und den aktiven Schlammbereichen ermittelt wird, um nicht nur einen endgültigen Schätzwert E(%) zu erhalten, sondern auch Informationen über die Eigenschaften der gemessenen Biomasse zu gewinnen, und zwar gemäß der Parameter $f_c$, $y_o$ $\alpha$ und A, welche Auskunft über die Art der untersuchten Zellen geben, wobei $f_c$ die Frequenz ist, bei welcher der größte Wert für den Imaginärteil erhalten wird, wobei diese von der Art des Mikroorganismus abhängt, $y_o$ Auskunft über die Eigenschaften des gerührten Mediums gibt, $\alpha$ ein Parameter ist, der die Verteilung der Zellgrößen und -formen bei den Mikroorganismen betrifft, und A Auskunft über das abgesetzte Medium gibt.

11. Verfahren nach den vorhergehenden Ansprüchen, **dadurch gekennzeichnet, dass** aus der Messung der Impedanz in den beiden vorstehend erwähnten Bereichen des Mediums zusätzlich zum Schätzwert E(%) Informationen über die Eigenschaften der gemessenen Biomasse erhalten werden, und zwar gemäß der Parameter $f_c$, $y_o$ $\alpha$ und A, welche Auskunft über die Art der untersuchten Zellpopulation zum Vergleich mit den Werten anderer Kulturen gibt, so dass die generische Verteilung der Population abgeschätzt werden kann.

12. Sonde zur Messung von Biomasse in zwei Bereichen eines Bioreaktors, **dadurch gekennzeichnet, dass** sie zwei Messbereiche gleicher Größe umfasst, wobei ein Bereich mit einem Netzgewebe abgedeckt ist, welches für die untersuchten Zellen undurchlässig ist, während der andere Bereich in direktem Kontakt mit der zellhaltigen Kultur steht.

13. Behälter für Oberflächenzellkulturen, **dadurch gekennzeichnet, dass** er zwei Sätze von Messelektroden umfasst, wobei der Bereich, welcher den einen dieser Sätze umgibt, bioverträglich ist und der Bereich, welcher den anderen Elektrodensatz umgibt, biounverträglich ist.

**Revendications**

1. Procédé de mesure de la concentration et de la composition d'une biomasse, qui consiste à évaluer la concentration cellulaire dans la culture en mesurant l'impédance électrique du milieu dans la biomasse à plusieurs fréquences comprises entre 1 kHz et 20 MHz,
**caractérisé en ce que** l'impédance électrique est mesurée dans deux régions du milieu, l'une étant exempte de cellules et l'autre contenant des cellules, les deux mesures étant réalisées avec des ensembles d'électrodes identiques auxquelles sont reliés deux niveaux frontaux du système de mesure : à partir de ces mesures de la zone exempte de cellules sont obtenues des informations sur les variations de la conductivité du milieu extra-cellulaire ($\sigma_e$), lesquelles informations sont utilisées pour corriger l'évaluation de la concentration cellulaire de la culture dans la région contenant des cellules.

2. Procédé selon la revendication 1, **caractérisé en ce que** la correction de l'évaluation de la concentration cellulaire basée sur la variation de la conductivité du milieu extra-cellulaire, $\sigma_\varepsilon$, est effectuée selon l'expression :

$$E_c(\%) = E(\%) \cdot 3 \frac{k}{2+k} ; k = \frac{\sigma_i}{\sigma_e}$$

où la conductivité du milieu intracellulaire ($\sigma_i$) peut être considérée comme constante pour un micro-organisme donné.

3. procédé selon la revendication 1, **caractérisé en ce qu'**une correction de l'évaluation de la concentration cellulaire est obtenue à partir d'une courbe ce calibrage obtenue à partir de données expérimentales, en faisant varier de manière artificielle la conductivité du milieu extra-cellulaire ($\sigma_c$).

4. Procédé selon la revendication 1, **caractérisé en ce que** la région exempte de cellules et la région contenant des cellules se trouvent dans le même réacteur ou cellule de culture, la région exempte de cellules étant séparée, de la culture par un grillage ayant une ouverture suffisamment étroite.

5. Procédé selon la revendication 1, **caractérisé en ce que** la séparation entre la région exempte de cellules et la région contenant des cellules est obtenue par sédimentation.

6. Procédé selon la revendication 1, **caractérisé en ce que** la région exempte de cellules est obtenue en déposant une substance non biocompatible sur la région de référence, ou par un traitement qui rend cette région de référence non biocompatible.

7. Procédé selon la revendication 1, **caractérisé en ce que** les régions contenant des cellules et exempte de cellules sont obtenues à partir d'un échantillon de culture qui est divisé en deux échantillons égaux, lesquels sont ensuite introduits dans des récipients identiques comportant des ensembles d'électrodes identiques, les cellules contenues dans l'un des échantillons étant détruites par ultrasons, rayonnement ultraviolet, des produits chimiques, traitement thermique (températures élevées) ou application de signaux électriques haute-tension.

8. Procédé selon les revendications ci-dessus, **caractérisé en ce que**, pour comparer et/ou traiter les mesures effectuées dans les deux régions, une mesure individuelle est réalisée dans chaque région en utilisant un niveau frontal pour chaque ensemble d'électrodes, ou en multiplexant un simple niveau frontal entre les deux ensembles d'électrodes de chaque région.

9. Procédé selon les revendications ci-dessus, **caractérisé en ce que**, pour comparer et/ou traiter les mesures effectuées avec les sondes, une mesure de différence directe est réalisée des tensions dans les électrodes de détection de chaque sonde, en les reliant selon un agencement de pont de Wheatstone ou de Kelvin ou similaire.

**10.** Procédé selon la revendication 5, **caractérisé en ce que** la sédimentation a lieu dans un tube de mesure dans lequel est introduit un volume prédéfini de vase, sa vitesse de sédimentation est mesurée et, pendant la sédimentation et après celle-ci, son impédance électrique est mesurée par comparaison entre les régions de vase actives et surnageantes, obtenant une évaluation finale E(%), ainsi que les propriétés de la biomasse mesurée selon les paramètres $f_c$, $y_o$, $\alpha$ et A, lesquels fournissent des informations sur le type de cellules étudiées, où : $f_c$ est la fréquence à laquelle la valeur de part imaginaire maximale est obtenue, et qui dépend du type de micro-organisme, $y_o$ donne des informations sur les propriétés du milieu agité ; $\alpha$ est un paramètre lié à la dispersion de tailles et formes de cellule pour les micro-organismes ; et A donne des informations concernant le milieu sédimenté.

**11.** Procédé selon les revendications précédentes, **caractérisé en ce que**, d'après la mesure de l'impédance dans les deux régions de milieu précitées, peuvent être obtenues, en plus de l'évaluation E (%), les propriétés de la biomasse mesurée selon les paramètres $f_c$, $y_o$, $\alpha$ et A, lesquels fournissent des informations sur le type de population de cellule étudiée pour comparaison à des valeurs d'autres cultures, permettant d'évaluer la répartition générique de la population.

**12.** Sonde permettant de mesurer une biomasse dans deux régions d'un bioréacteur, **caractérisée en ce qu'**elle comprend deux régions de mesure de taille égale, une région étant recouverte d'un grillage qui empêche le passage des cellules en cours d'analyse, tandis que l'autre région est en contact direct avec la culture contenant des cellules.

**13.** Cellule pour cultures de cellules de surface, **caractérisée en ce qu'**elle comprend deux ensembles d'électrodes de mesure, la région située autour d'un de ces ensembles étant biocompatible et la région située autour de l'autre ensemble d'électrodes étant non biocompatible.

**Re**

**Ri**

**C**

FIG. 1

FIG. 2

3

6

8

2

Vm

7

4

Im

5

A

2

3

1

6

8

Vmr

7

4

Imr

B

FIG. 3

12

13

15

14

Vm

14

12

13

11

10

16

16

19

9

17

18

17

A

23

24

22

27

26

25

20

21

FIG. 4

FIG. 5

FIG. 6

FIG. 7

41

40

42

39

FIG. 8

## Population Distribution

43    44    45    46

Type of celule

FIG. 9